(19)

Europäisches
Patentamt

European
Patent Office

Office européen
des brevets

(11) **EP 3 249 386 A1**

(12) **EUROPÄISCHE PATENTANMELDUNG**

(43) Veröffentlichungstag:
**29.11.2017  Patentblatt 2017/48**

(21) Anmeldenummer: **17172345.5**

(22) Anmeldetag: **23.05.2017**

(51) Int Cl.:
**G01N 21/59** *(2006.01)*       **G01N 21/78** *(2006.01)*
**G01N 33/18** *(2006.01)*       **G01F 23/20** *(2006.01)*
**G01N 21/11** *(2006.01)*

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**MA MD**

(30) Priorität: **24.05.2016   DE 102016208962**

(71) Anmelder: **AXAGARIUS GmbH & Co. KG
52355 Düren (DE)**

(72) Erfinder:
• **Meusel, Markus
  52146 Würselen (DE)**
• **Prokisch, Christian
  52355 Düren (DE)**

(74) Vertreter: **Jostarndt Patentanwalts-AG
Philipsstrasse 8
52068 Aachen (DE)**

Bemerkungen:
Die Bezugnahmen auf die Zeichnung(en) Nr. 7 gelten
als gestrichen (R. 56(4) EPÜ).

(54) **PHOTOMETER MIT QUANTITATIVER VOLUMENERFASSUNG**

(57)    Die vorliegende Erfindung betrifft ein Photometer, das eine Wägevorrichtung zum Wiegen der Messküvette zur quantitativen Erfassung des Flüssigkeitsvolumens in einer Messküvette umfasst. Die Erfindung betrifft zudem ein Verfahren zur photometrischen Konzentrationsbestimmung in einer flüssigen Probe, bei der die quantitative Erfassung des Flüssigkeitsvolumens zur Korrektur der photometrisch gemessenen Konzentration verwendet wird.

**EP 3 249 386 A1**

**Beschreibung**

Die vorliegende Erfindung betrifft ein Photometer, das eine Vorrichtung zur quantitativen Erfassung des Flüssigkeitsvolumens in einer Messküvette umfasst. Die Erfindung betrifft zudem ein Verfahren zur photometrischen Konzentrationsbestimmung in einer flüssigen Probe, bei der die quantitative Erfassung des Flüssigkeitsvolumens zur Korrektur der photometrisch gemessenen Konzentration verwendet wird.

Lösungen gefärbter Stoffe erscheinen umso intensiver, je höher die Konzentration der Stoffe in der Lösung ist. Die Farbe entsteht durch Absorption bestimmter Wellenlänge durch die gelösten Substanzen.

In der Photometrie wird diese Absorption sehr präzise gemessen und daraus die Konzentration der gelösten Substanz ermittelt. Üblicherweise wird Licht einer bestimmten Wellenlänge durch die Probe geleitet, dem Licht wird die Ausgangsintensität $I_0$ zugeordnet. Das Licht hinter der Probe (nach Absorption durch in der Lösung vorhandene Bestandteile der Probe) wird als Intensität I gemessen. Der Quotient aus I und $I_0$ wird als Transmission bezeichnet. Ausgehend von der Transmission wird eine weitere wichtige Größe, die dimensionslose Extinktion, bestimmt. Die Extinktion wird üblicherweise mit einem Photometer bestimmt, bei dem die in einer Messküvette vorliegende Probe von einem Lichtstrahl durchquert wird und mittels eines hinter der Küvette angeordneten Detektors die Abschwächung der Intensität gemessen wird.

In der Analytik interessiert zumeist jedoch nicht direkt die Extinktion, sondern die Konzentration der gelösten Substanz. Diese kann jedoch aus der Extinktion $E$ nach dem Gesetz von Lambert-Beer einfach bestimmt werden. Hier gilt: $E = \varepsilon \times c \times d$, mit $\varepsilon$ als Extinktionskoeffizienten, dem Durchmesser der Küvette im Strahlengang $d$ und der Stoffkonzentration $c$. Mit bekannten Werten für $\varepsilon$ und $d$ kann damit leicht über die Extinktion die Stoffkonzentration $c$ ermittelt werden.

Hiermit wird klar, dass der gemessene Extinktionswert unmittelbar ein Maß für die Konzentration darstellt. Üblicherweise wird zunächst eine Kalibration mit bekannten Konzentrationen einer Substanz erstellt und dann anhand der Extinktion unbekannter Proben deren Konzentration berechnet. Bei der praktischen Durchführung kann z.B. eine Reaktionslösung in der Küvette vorgelegt werden. Wird Probe hinzugegeben, so reagieren Bestandteile der Reaktionslösung mit den Probenbestandteilen und es erfolgt eine konzentrationsabhängige Farbreaktion. Dabei ist es sehr wichtig, dass das Volumen der Probe möglichst exakt zuzugeben wird, da diese ja durch die vorgelegte Reaktionslösung verdünnt wird.

In vielen Fällen ist nun eine Probenvorbehandlung vor der eigentlichen photometrischen Messung notwendig. Ein solcher Fall liegt beispielsweise bei der photometrischen CSB-Bestimmung vor. Der CSB (chemischer Sauerstoffbedarf) ist ein Maß für die Belastung von Wasser mit organischen Substanzen. Dieser Parameter liefert beispielsweise Anhaltspunkte für die Reinigungsleistung (Effizienz) von Kläranlagen und wird im Zu- und Ablauf von Kläranlagen gemessen.

Bei diesem Parameter kann es aber zu Störungen der Messung durch im Wasser vorhandene Chloridionen kommen. Üblicherweise wird die Störung durch Chlorid dadurch eliminiert, dass zur Reaktionslösung Quecksilbersalze gegeben werden, z.B. Quecksilbersulfat. Aus Gründen des Umweltschutzes möchte man gerne auf das Quecksilber verzichten. In einem verbesserten Verfahren wird das Chlorid im Rahmen einer Probenvorbereitung durch Fällung z.B. mit Silbersulfat entfernt. Der Anwender zieht dazu üblicherweise eine Wasserprobe in eine Kartusche, in der sich Silbersulfat befindet. Dann wird die Kartusche geschüttelt und die klare, vom Chlorid befreite Lösung in ein Reaktionsglas gegeben. Aus diesem Gefäß wird dann eine definierte Menge (z.B. exakt 2 mL) in eine Küvette mit Reaktionslösung für die CSB Bestimmung gegeben. Um Verdünnungsfehler zu vermeiden, ist die exakte Zugabe an dieser Stelle kritisch.

Da das Volumen exakt bestimmt werden muss, ist eine Zugabe direkt aus der Kartusche nicht möglich. Die Verwendung eines weiteren Gefäßes und die nachfolgende exakte Dosierung mit einer Pipette sind unumgänglich. Dies Verfahren ist somit aufwändig und fehleranfällig, insofern es einen Pipettierschritt als essentiellen Schritt des Verfahrens beinhaltet.

Selbst bei Verwendung einer Pipette können Pipettierfehler auftreten, die dann unweigerlich zu einer fehlerhaften Konzentrationsbestimmung führen.

Es besteht daher ein Bedarf an verbesserten Verfahren und Vorrichtungen zur photometrischen Bestimmung von Probenkonzentrationen.

Aufgabe der Erfindung ist es daher, ein Photometer bzw. ein photometrisches Testverfahren dahingehend zu verbessern, dass sie bezüglich mindestens einer der oben genannten Nachteile verbessert wird.

Diese Aufgabe wird gemäß der Erfindung durch den Gegenstand des Hauptanspruchs gelöst. Spezifische Ausführungsformen der Erfindung sind Gegenstand der zusätzlichen abhängigen oder unabhängigen Ansprüche.

**Zusammenfassung der Erfindung**

In einem ersten Aspekt stellt die Erfindung ein Photometer zur Messung einer flüssigen Probe in einer Messküvette bereit, das eine Küvettenaufnahme und eine Vorrichtung zur quantitativen Erfassung des Flüssigkeitsvolumens innerhalb der Messküvette umfasst.

Das erfindungsgemäße Photometer vereinigt mehrere entscheidende Vorteile gegenüber den aus dem Stand der Technik bekannten Photometern.

Wie die Erfinder festgestellt haben, stellt eine im Photometer integrierte Vorrichtung zur Volumenerfassung eine einfache und effektive Möglichkeit bereit, das Flüssigkeitsvolumen in der Messküvette zu erfassen, und damit entsprechend eine Volumenkorrektur durchzuführen. Damit kann basierend auf der direkt über die Extinktion ermittelten Konzentration $C_{gemessen}$ eines vermessenen Probenbestandteils eine korrigierte Konzentration $C_{korrigiert}$ berechnet werden.

Da die Messvorrichtung für das Flüssigkeitsvolumen ein integraler Bestandteil des Photometers ist, wird kein zusätzliches Gerät benötigt.

Ein besonderer Vorteil ist, dass sogar ganz auf eine Pipettiervorrichtung verzichtet werden kann. Der Benutzer kann beispielsweise die Probenflüssigkeit mittels einer an der Messküvette vorhandenen Markierung zugeben, ohne das Volumen exakt bestimmen zu müssen. Durch die im Photometer integrierte Messvorrichtung wird das Flüssigkeitsvolumen dann exakt erfasst und direkt zur Berechnung der photometrischen Daten verwendet.

Dies ist gerade auch bei Messungen außerhalb eines Laboratoriums, wie beispielsweise bei Feldmessungen oder Messungen in Kläranlagen von Vorteil, da neben einem portablen Photometer lediglich die mit Reaktionslösung vorbereiteten Messküvetten benötigt werden.

Das erfindungsgemäße Photometer bietet weiterhin den Vorteil, dass die Messwerte mit Hilfe einer in dem Photometers vorhandenen Datenverarbeitungseinheit ausgewertet können und zur Korrektur anderer Messwerte verwendet werden können.

Die quantitative Erfassung kann durch verschiedenste Verfahren erfolgen, wie beispielsweise durch eine Wägevorrichtung, optische Messverfahren oder auch sonographische Messverfahren. Der Fachmann kann hier entsprechend den apparativen oder analytischen Anforderungen das geeignete Messverfahren auswählen.

Gerade Messvorrichtungen, die mittels einer Wägung oder einer optischen Detektion arbeiten, sind apparativ einfach in ein Photometer integrierbar. Da ein Photometer eine Küvettenaufnahme besitzen muss, kann diese als Wägeplattform ausgestaltet werden, so dass die photometrische Messung und die Gewichtsbestimmung zusammen durchgeführt werden können und damit eine einfache, schnelle und fehlerfreie Messung ermöglicht wird.

Optische Messvorrichtungen lassen sich entsprechend einfach integrieren, insofern sie die bereits vorhandenen Komponenten zur Lichterzeugung, Lichtweiterleitung und Lichtdetektion ausnutzen können.

Die Messvorrichtung für das Flüssigkeitsvolumen kann auch vorteilhaft für andere Zwecke eingesetzt werden. So können Messproben in kontrollierter Weise verdünnt werden, bzw. die Verdünnung quantitativ erfasst werden. Hierfür kann die verdünnungs-assoziierte Abnahme der Extinktion einer Probensubstanz herangezogen werden.

Zudem kann die Messvorrichtung für das Flüssigkeitsvolumen dazu dienen, Fehler zu vermeiden, so kann eine zu geringe Befüllung der Messküvette, bei der z.B. der Lichtstrahl über der Flüssigkeit oder im Bereich des streuenden Meniskus verläuft, detektiert werden und in einem Warnhinweis an den Benutzer resultieren.

Weiterhin erlaubt die Messvorrichtung, insbesondere bei Verwendung einer Wägevorrichtung völlig neue photometrische Messverfahren. So können zusätzliche feste oder flüssige Substanzen in kontrollierter Weise der Messküvette zugegebenen, ohne dass eine externe Waage notwendig ist.

Diese Messvorrichtung erleichtert auch die Erstellung einer Reaktionskinetik, insofern die durch die Messvorrichtung genau zeitlich erfassbare Zugabe eines "Startreagenzes" die kinetische Messung initiiert.

Da einfache Messvorrichtungen verwendbar sind, wie bspw. elektronische Waagen kann das erfindungsgemäße Photometer auf einfache Weise und zudem kostengünstig hergestellt werden.

Das erfindungsgemäße Photometer kann ohne Mehraufwand in die bereits etablierten Testsysteme integriert werden und sogar eine Wägevorrichtung ersetzen. Es stellt damit ein multifunktionales Gerät dar. Als solches kann es beispielweise mit einer Rühreinrichtung und/oder einer Temperiereinrichtung kombiniert werden.

## Ausführliche Beschreibung der Erfindung

Erfindungsgemäß weist das Photometer eine Vorrichtung zur quantitativen Erfassung des Flüssigkeitsvolumens innerhalb der Messküvette auf. Da es sich um nicht um eine qualitative Erfassung, sondern um eine quantitative Erfassung des Füllvolumens handelt, kann der gemessene Wert zur Volumenkorrektur der Messung dienen.

Dem Fachmann stehen zahlreiche Möglichkeiten zur Bestimmung des Flüssigkeitsvolumens zur Verfügung, die er unter Berücksichtigung des Messaufwands, der Messgenauigkeit und des Preises auswählen wird.

In einer Ausführungsform der Erfindung ist bei dem Photometer die Vorrichtung zur Bestimmung des Flüssigkeitsvolumens ausgewählt aus der Gruppe enthaltend:

    a) Wägevorrichtung zum Wiegen der Messküvette;
    b) Sonographische Messeinrichtung zur Bestimmung der Füllhöhe der Messflüssigkeit in der Messküvette;

c) Optische Messeinrichtung zur Bestimmung der Füllhöhe der Messflüssigkeit in der Messküvette; oder
d) Bildaufzeichnungseinrichtung zur Erfassung der Füllhöhe der Messflüssigkeit in der Messküvette.

In einer weiteren Ausführungsform kann das erfindungsgemäße Photometer mehrere der oben aufgeführten konkreten Messvorrichtungen umfassen, so z.B. eine sonographische und eine optische Messeinrichtung oder eine Wägevorrichtung und ein Bildaufzeichnungssystem.

Dem Fachmann ist zudem bewusst, dass auch andere als die oben aufgeführten Methoden zur Erfassung der Füllhöhe (wie etwa durch Leitfähigkeitsmessung) prinzipiell auch nach dem erfindungsgemäßen Verfahren eingesetzt werden können.

Die quantitative Erfassung des Flüssigkeitsvolumens durch eine Wägevorrichtung stellt beachtlicherweise eine indirekte Messmethode dar. Basierend auf dem gemessenen Gewicht muss über die Dichte der Flüssigkeit auf das vorhandene Flüssigkeitsvolumen zurückgeschlossen werden. Diese Berechnung erfolgt anhand der folgenden Formel:

$$V_F = \frac{M_F}{\rho}$$

Hierbei ist $V_F$ das Flüssigkeitsvolumen, $M_F$ die durch die Wägevorrichtung gemessene Masse und $\rho$ die Dichte der Messflüssigkeit.

In einer bevorzugten Ausführungsform der Erfindung wird die Dichte gleich 1,0 g·cm$^{-3}$ gesetzt und die in Gramm (g) gemessene schwere Masse entspricht dem Volumen in ml. Diese Prämisse sollte für die meisten im Laborbereich verwendeten Flüssigkeiten zu akzeptablen Ergebnissen führen, da deren Dichte nur unwesentlich von der Dichte reinen Wassers mit 1,0 g·cm$^{-3}$ abweicht.

In einer weiteren Ausführungsform ist bei Verwendung von Reaktions- oder Probenlösungen mit stark hiervon abweichender Dichte im Photometer (Dichte ungleich 1,0 g·cm$^{-3}$) vorgesehen, dass die tatsächliche Dichte in der Berechnung verwendet wird. Alternativ kann ein "Dichteoffset" hinterlegt sein, um zusätzlich eine Dichteoffsettkorrektur als Funktion der Volumendifferenz durchzuführen. Damit können auch diese Lösungen mit hoher Genauigkeit vermessen werden.

In einer Ausführungsform der Erfindung wird bei der photometrischen Messung eine Probe, die bevorzugt eine flüssige Lösung ist, zu einer vorgelegten Probe, die bevorzugt eine Reaktionslösung ist, hinzugegeben. Hierbei ist es vorteilhaft das Gewicht der Messküvette inklusive dieser vorgelegten Probe als Blindwert im Photometer zu hinterlegen, so dass das Photometer nur das Gewicht der zugegebenen Probe erfasst und hieraus dessen Volumen berechnet.

In einer alternativen Ausführungsform wird die Messküvette ohne Probenlösung gewogen, dann eine Wägung nach Zugabe der Probenlösung durchgeführt und durch Differenzbildung das Volumen der Probenlösung quantitativ erfasst. Eine Wägung der Ausgangslösung bietet den Vorteil, dass hierdurch eine zusätzliche Kontrollmöglichkeit einer ordnungsgemäßen Messung erfolgen kann.

In einer bevorzugten Ausführungsform der Erfindung weist die Wägevorrichtung des Photometers eine Tara-Funktion auf, so dass zuerst die Messküvette ohne Probenlösung gewogen wird, das erhaltene Gewicht hierbei zu Null gesetzt wird und anschließend das Gewicht der Probe erfasst wird.

Das erfindungsgemäße Verfahren ist auch ideal für die Vermessung von festen Substanzen. So kann beispielsweise eine als Festphase bereitgestellte Reaktionssubstanz vorgelegt werden, die dann mit der flüssigen Probenlösung vermischt wird und nach Auflösen oder Absetzen der Reaktionssubstanz in der Messküvette die Gesamtlösung photometrisch vermessen werden. Hier dient die Volumenbestimmung dazu, dass Gesamtvolumen nach Auflösung oder Absetzen der festen Phase zu ermitteln. Alternativ kann auch die Reaktionssubstanz als Flüssigkeit in der Messküvette vorgelegt sein und nach Zugabe einer festen Probe die resultierende Lösung photometrisch vermessen werden.

Dem Fachmann stehen zahlreiche Wägevorrichtungen zur Verwendung im Photometer zur Verfügung. In einer bevorzugten Ausführungsform ist die Wägevorrichtung zum Wiegen der Messküvette ausgewählt aus der Gruppe enthaltend elektronische Waage, elektromechanische Waage, elektrische Waage, magnetische Waage und Präzisionswaage.

Erfindungsgemäß kann die quantitative Erfassung des Flüssigkeitsvolumens auch durch eine sonographische Messeinrichtung erfolgen. Diese ist zweckmäßigerweise so eingerichtet, dass sie die Füllhöhe der Messflüssigkeit in der Messküvette erfasst.

In einer bevorzugten Ausführungsform handelt es sich bei der sonographischen Messeinrichtung um eine Ultraschallsonde oder einen Ultraschallkopf, der Ultraschallwellen aussendet und empfängt.

In einer weiterhin bevorzugten Ausführungsform ist die Ultraschallsonde oberhalb der Messküvette positioniert und misst so durch die Ultraschallreflexion an der Flüssigkeitsoberfläche den Abstand zwischen Ultraschallsonde und Flüssigkeitsoberfläche, wodurch indirekt auch das Volumen der Flüssigkeit erfasst wird.

In einer alternativen Ausführungsform ist die Ultraschallsonde unterhalb der Messküvette angebracht und misst die Höhe der Flüssigkeitssäule. Eine solche Anordnung hat den Vorteil, dass die Ultraschallsonde zudem für eine Ultraschall-vermittelte Vermischung der Proben verwendet werden kann.

Erfindungsgemäß kann die quantitative Erfassung des Flüssigkeitsvolumens auch durch eine optische Messeinrichtung erfolgen. Diese ist zweckmäßigerweise so eingerichtet, dass sie die Füllhöhe der Messflüssigkeit in der Messküvette erfassen.

Die optische Messeinrichtung kann sich hierbei für die Volumenmessung unterschiedlichsten Verfahren bedienen. So kann die optische Messeinrichtung das Flüssigkeitsvolumen quantitativ mittels Transmission, Absorption, Reflexion oder Polarisation erfasst.

In einer Ausführungsform umfasst die optische Messeinrichtung hierbei mindestens eine Lichtquelle und mindestens einen Detektor.

Die mindestens eine Lichtquelle kann erfindungsgemäß ausgewählt sein aus der Gruppe enthaltend Glühlampe, Nernstlampe, Leuchtstofflampe, Gasentladungslampe, Leuchtdiode, Elektrolumineszenzfolie und Laser. Bevorzugt ist hierbei die Verwendung eines Lasers, der durch die scharfe Strahlbündelung eine genaue Messung erlaubt.

Der mindestens eine Detektor kann erfindungsgemäß ausgewählt sein aus der Gruppe aufweisend Photozelle, Photomultiplier, Fotowiderstand, Photodiode, Fototransistor, CCD-Sensor und Halbleiterelement, wobei die Verwendung einer Photodiode bevorzugt ist.

In einer bevorzugten Ausführungsform erfasst die optische Messeinrichtung die Weglänge von der Lichtquelle zur Flüssigkeitsoberfläche und/oder die Weglänge durch die Flüssigkeitssäule innerhalb der Messküvette. Hierzu ist der mindestens eine Lichtquelle oder die mindestens eine Detektor bevorzugt oberhalb bzw. unterhalb der Messküvette angeordnet.

Alternativ kann die Lichtquelle auch vor der Messküvette angeordnet sein, so dass die Lichtstrahlen die Messküvette mit der Messflüssigkeit seitlich durchstrahlen und das transmittierte oder reflektierte Licht von einem oder mehreren entsprechend positionierten Detektoren zur Bestimmung des Flüssigkeitsvolumens dient. Hierzu kann auch die interne, d.h. zur photometrischen Messung verwendete Lichtquelle des Photometers eingesetzt werden.

In einer weiteren Ausführungsform wird die Messküvette im Strahlengang vertikal verschoben, so dass die Lage der Flüssigkeitsoberfläche durch Reflexion/Streuung im Bereich des Meniskus oder eine stark veränderte Transmission oberhalb der Flüssigkeit nachweisbar ist. Diese Ausführungsform hat den Vorteil, dass bei dieser Messung auch die für die photometrische Messung bereits Komponenten wie Lichtquelle, Küvettenaufnahme und Detektor verwendet werden können.

Diese Verschiebung kann beispielsweise durch eine vertikal verschiebbare Küvettenaufnahme erfolgen. In bevorzugter Weise wird für die Verschiebung ein unterhalb der Messküvette angeordneter Aktuator verwendet.

Erfindungsgemäß kann die quantitative Erfassung des Flüssigkeitsvolumens auch durch eine Bilderfassungseinrichtung erfolgen. Diese ist zweckmäßigerweise so positioniert, dass das hierbei erfasste Bild die Füllhöhe der Messflüssigkeit in der Messküvette wiedergibt.

Mögliche Bilderfassungseinrichtungen sind beispielsweise Digitalkamera, CCD-Kamera oder Videokamera. Gerade im Bereich der miniaturisierten Kameratechnologie sind preisgünstige Geräte mit sehr guter Aufnahmequalität verfügbar. Zudem könnte die interne Lichtquelle des Photometers zur photographisch notwendigen Beleuchtung der Messküvette dienen, so dass hier keine zusätzliche Belichtung und kein Blitzlicht erforderlich ist. Eine solche Bilderfassung der jeweiligen Messküvette, die bevorzugterweise in der Küvettenaufnahme erfolgen sollte, kann zudem auch zur Qualitätssicherung dienen, indem sie über die photometrische Messung hinaus zur Dokumentation der jeweiligen Messbedingungen dienen könnte.

Erfindungsgemäß kann die Messvorrichtung zur Erfassung des Flüssigkeitsvolumens in dem Photometer an beliebiger Stelle positioniert sein.

In vorteilhafter Weise wird bei dem Photometer die quantitative Erfassung des Flüssigkeitsvolumens an der in der Küvettenaufnahme befindlichen Messküvette vorgenommen. Zweckmäßigerweise ist hierzu die Vorrichtung zur quantitativen Erfassung des Flüssigkeitsvolumens ein Bestandteil der Küvettenaufnahme oder sie ist mit der Küvettenaufnahme verbunden.

In einer weiteren Ausführungsform weist das Photometer zusätzlich eine Datenverarbeitungseinheit zur Auswertung der Messergebnisse der Messvorrichtung auf. Da Photometer üblicherweise eine Datenverarbeitungseinheit zur Auswertung der photometrischen Messergebnisse aufweisen, kann diese Datenverarbeitungseinheit in bevorzugter Weise auch die Berechnung des Flüssigkeitsvolumens durchführen und dann in einem Folgeschritt die Berechnung der korrigierten Konzentrationen anhand der photometrisch gemessenen Konzentration unter Berücksichtigung des Flüssigkeitsvolumens durchführen.

In einer besonderen Ausführungsform erfolgt bei dem Photometer die Messung der flüssigen Probe anhand einer vorgelegten Reaktionslösung und einer durch Volumenkorrektur erfassten, separat hinzugegebenen Probenlösung. Hierbei erfolgt die Auswertung der Messergebnisse, bevorzugt dann in der Datenverarbeitungseinheit des Photometers, durch eine Berechnung gemäß der folgenden Formel oder durch eine darauf basierenden Wertetabelle:

$$\frac{(Vr + Vprobe + \Delta V) \times Vprobe}{(Vprobe + \Delta V) \times (Vr + Vprobe)} \times E_{gemessen} = E_{korrigiert}$$

Hierbei ist *Vr* das Volumen der vorgelegten Reaktionslösung, *Vprobe* das geforderte Volumen der Probenlösung, *ΔV* der gemäß quantitativer Volumenerfassung ermittelte Volumenfehler der Probe als Abweichung von dem geforderten Volumen *Vprobe, E_{gemessen}* die photometrisch ermittelte Extinktion und *E_{korrigiert}* die korrigierte Extinktion.

Wird je nach Test eine Abnahme der Extinktion gemessen (wie z.B. bei einigen CSB Testverfahren), dann muss die Extinktion des Nullwertes berücksichtigt werden. Die Abhängigkeit der Extinktion des Nullwertes vom Probenvolumen kann berechnet werden, sollte aber in der Praxis experimentell ermittelt und im Gerät, und hier bevorzugt in der Datenverarbeitungseinheit hinterlegt werden. In der o.g. Formel muss dann "E gemessen" durch "E gemessen - $E_0(v)$" ersetzt werden, wobei $E_0(v)$ die Extinktion des Nullwertes beim Probenvolumen v ist. Es kann darüber hinaus weitere volumenabhängige Korrekturfaktoren geben, die im Einzelfall bei der Korrektur berücksichtigt werden müssen.

Beachtlicherweise kann mit dem erfindungsgemäßen Photometer nicht nur das Volumen der Probe *Vprobe* + *ΔV* quantitativ erfasst werden, sondern auch das Volumen *Vr* der vorgelegten Reaktionslösung, so dass zusammen mit der photometrisch bestimmten Konzentration alle Messdaten mit nur einem Gerät und darüber hinaus zum Zeitpunkt der Messung erfasst werden. Damit ist das Risiko einer fehlerhaften Messung extrem reduziert.

In einer bevorzugten Ausführungsform weist das Photometer zusätzlich eine Anzeigeeinrichtung zur Darstellung der jeweiligen Messdaten und der berechneten Werte und bevorzugt der korrigierten Probenkonzentration auf.

In einer weiteren Ausführungsform kann man die Volumenfehler durch eine Funktion korrigieren, die zuvor ermittelt und z.B. in der Auswerteeinheit als Formel hinterlegt sein kann. Diese Formel kann so hinterlegt werden, dass entweder auf der Ebene der Extinktion oder auf der Ebene der Konzentration korrigiert wird.

Erfindungsgemäß können als Photometer unterschiedlichste Photometer eingesetzt werden. Beispielhaft seien erwähnt: Spektralphotometer, Filterphotometer, und LED-Photometer. In bevorzugter Weise handelt es sich bei dem erfindungsgemäßen Photometer um ein portables Photometer.

In einer weiteren Ausführungsform wird bei dem erfindungsgemäßen Photometer die Bestimmung des Flüssigkeitsvolumens zur Korrektur eines photometrischen Messwerts verwendet, wobei die Korrektur durch verschiedene Verfahren erfolgen kann, die entweder auf im Photometer hinterlegten Formeln oder Korrekturfaktoren basieren oder auf der empirischen Berechnung von Korrekturfaktoren anhand von Funktionen oder Funktionsscharen beruhen. Folgende Korrekturverfahren seien hier beispielhaft beschrieben:

In einem ersten Verfahren wird eine allgemeine Formel zur Korrektur der Messwerte bereitgestellt.

Alternativ kann die Funktion zu Berechnung eines Korrekturfaktors empirisch ermittelt werden, wobei in die Berechnung des Korrekturfaktors nur das tatsächlich pipettierte Volumen oder die Abweichung zum korrekten Wert eingehen.

In einer weiteren Ausführungsform wird eine Schar von Funktionen zur Berechnung eines Korrekturfaktors empirisch ermittelt, wobei in die Berechnung des Korrekturfaktors neben dem tatsächlich pipettierten Volumen auch die tatsächlich gemessene Extinktion eingeht.

In einer weiteren Ausführungsform wird eine sogenannte Korrekturmatrix empirisch ermittelt, wobei mit Hilfe der tatsächlich ermittelten Extinktion und dem tatsächlich pipettierten Volumen bzw. der Abweichung vom korrekten Volumen der Korrekturfaktor abgelesen wird.

Diese vorab beschriebenen Korrekturverfahren sind hiermit nicht nur im Rahmen des Photometers beansprucht und offenbart sondern auch als eigenständige Verfahren zur Korrektur eines Messwerts basierend auf der Bestimmung des Flüssigkeitsvolumens innerhalb der Messküvette.

In einem zweiten Aspekt stellt die Erfindung ein Verfahren zur photometrischen Konzentrationsbestimmung von mindestens einem Probenbestandteil in einer flüssigen Probe bereit, wobei das Verfahren die folgenden Schritte umfasst:

a) Vorlegen einer Reaktionslösung in der Messküvette mit dem Volumen Vr;

b) Optional die quantitative Erfassung des Gewichts oder des Volumens der Reaktionslösung als Blindwert, bevorzugt im Photometer durchgeführt;

c) Zugabe der Probenlösung und Vermischung mit der Reaktionslösung;

d) Optional eine Dichtekorrektur, optional als Funktion der Volumendifferenz;

e) Quantitative Erfassung des Flüssigkeitsvolumens der Lösung aus Schritt c) mit Hilfe einer Wägevorrichtung, sonographischen Messvorrichtung, optischen Messvorrichtung oder Bildaufzeichnungsvorrichtung, bevorzugt innerhalb eines Photometers;

f) Photometrische Bestimmung der Konzentration der Probe in der Lösung aus Schritt c).

g) Berechnung einer korrigierten Konzentration anhand des in Schritt e) quantitativ erfassten Volumens.

Dieses vorab geschilderte Verfahren wird in bevorzugter Weise mit einem Photometer gemäß der Erfindung durchgeführt.

Bei dem erfindungsgemäßen Verfahren zur photometrischen Konzentrationsbestimmung einer flüssigen Probe wird die Berechnung der korrigierten Konzentration bevorzugt anhand der folgenden Formel durchgeführt:

$$\frac{(Vr + Vprobe + \Delta V) \times Vprobe}{(Vprobe + \Delta V) \times (Vr + Vprobe)} \times E_{gemessen} = E_{korrigiert}$$

wobei *Vr das* Volumen der vorgelegten Reaktionslösung, *Vprobe* das geforderte Volumen der Probenlösung, $\Delta V$ der Volumenfehler der Probe, $E_{gemessen}$ die photometrisch ermittelte Extinktion und $E_{korrigiert}$ die korrigierte Extinktion ist.

In spezieller Weise umfasst die vorliegende Erfindung einer der folgenden Ausführungsformen:

Ausführungsform 1: Ein Photometer zur Messung einer Messflüssigkeit in einer Messküvette umfassend eine Küvettenaufnahme und eine Vorrichtung zur quantitativen Erfassung des Flüssigkeitsvolumens innerhalb der Messküvette.

Ausführungsform 2: Photometer gemäß Ausführungsform 1, dadurch gekennzeichnet, dass die Vorrichtung zur quantitativen Erfassung des Flüssigkeitsvolumens eine der folgenden Vorrichtungen ist:

a) Wägevorrichtung zum Wiegen der Messküvette;
b) Sonographische Messeinrichtung zur Bestimmung der Füllhöhe der Messflüssigkeit in der Messküvette;
c) Optische Messeinrichtung zur Bestimmung der Füllhöhe der Messflüssigkeit in der Messküvette;
d) Bildaufzeichnungseinrichtung zur Erfassung der Füllhöhe der Messflüssigkeit in der Messküvette.

Ausführungsform 3: Photometer gemäß Ausführungsform 2, dadurch gekennzeichnet, dass die quantitative Erfassung des Flüssigkeitsvolumens durch die Wägevorrichtung anhand der folgenden Formel erfolgt:

$$V_F = \frac{M_F}{\rho},$$

wobei $V_F$ das Flüssigkeitsvolumen, $M_F$ *die* durch die Wägevorrichtung gemessene Masse und $\rho$ die Dichte der vermessenen Flüssigkeit ist, die bevorzugt gleich $1{,}0 \, \mathrm{g} \, / \, \mathrm{cm}^{-3}$ gesetzt wird, und wobei bei Zugabe der zu vermessenen Flüssigkeit zu einer vorgelegten Flüssigkeit mit stark abweichender Dichte im Photometer bevorzugt die Dichte korrigiert wird oder ein Dichteoffset hinterlegt ist, um zusätzlich eine Dichteoffsettkorrektur als Funktion der Volumendifferenz durchzuführen.

Ausführungsform 4: Photometer gemäß Ausführungsform 3, dadurch gekennzeichnet, dass das Gewicht oder das Volumen der vorgelegten Flüssigkeit als Blindwert vor Zugabe der zu vermessenen Flüssigkeit entweder gemessen wird und zur Korrektur des Blindwertes dient oder als Blindwert im Photometer hinterlegt ist.

Ausführungsform 5: Photometer gemäß Ausführungsform 2 bis 4, dadurch gekennzeichnet, dass die Wägevorrichtung zum Wiegen der Messküvette ausgewählt ist aus der Gruppe enthaltend elektronische Waage, elektromechanische Waage, elektrische Waage, magnetische Waage und Präzisionswaage.

Ausführungsform 6: Photometer gemäß Ausführungsform 2, dadurch gekennzeichnet, dass die sonographische Messeinrichtung eine Ultraschallsonde umfasst, wobei die Messeinrichtung bevorzugt die Weglänge von der Flüssigkeitsoberfläche zur Ultraschallsonde erfasst.

Ausführungsform 7: Photometer gemäß Ausführungsform 2, dadurch gekennzeichnet, dass die optische Messeinrichtung eine Lichtquelle umfasst, die bevorzugt eine Laserquelle ist, und wobei die Lichtquelle bevorzugt oberhalb der Flüssigkeitsoberfläche der Flüssigkeit positioniert ist und/oder die Messeinrichtung die Weglänge von der Lichtquelle zur Flüssigkeitsoberfläche und/oder die Weglänge durch die Flüssigkeitssäule innerhalb der Messküvette erfasst.

Ausführungsform 8: Photometer gemäß Ausführungsform 2, dadurch gekennzeichnet, dass die optische Messeinrichtung eine Lichtquelle aufweist, die zur quantitativen Volumenerfassung eine seitliche Durchstrahlung der Messküvette durchführt und bevorzugt der Lichtquelle entspricht, die zur photometrischen Messung der Messküvette benutzt wird.

Ausführungsform 9: Photometer gemäß Ausführungsform 2, dadurch gekennzeichnet, dass die optische Messeinrichtung das Flüssigkeitsvolumen quantitativ mittels Transmission, Absorption, Reflexion oder Polarisation erfasst.

Ausführungsform 10: Photometer gemäß Ausführungsform 2, dadurch gekennzeichnet, dass die Bildaufzeichnungseinrichtung eine Digitalkamera ist.

Ausführungsform 11: Photometer gemäß einem der Ausführungsformen 1 bis 10, dadurch gekennzeichnet, dass bei dem Photometer die quantitative Erfassung des Flüssigkeitsvolumens an der in der Küvettenaufnahme befindlichen Messküvette vorgenommen wird.

Ausführungsform 12: Photometer gemäß einem der Ausführungsformen 1 bis 11, dadurch gekennzeichnet, dass die Vorrichtung zur quantitativen Erfassung des Flüssigkeitsvolumen ein Bestandteil der Küvettenaufnahme ist.

Ausführungsform 13: Photometer gemäß einem der Ausführungsformen 1 bis 12, dadurch gekennzeichnet, dass das Photometer zusätzlich eine Datenverarbeitungseinheit zur Auswertung der Messergebnisse der Messvorrichtung aufweist.

Ausführungsform 14: Photometer gemäß einem der Ausführungsformen 1 bis 13, dadurch gekennzeichnet dass die Bestimmung des Flüssigkeitsvolumen zur Korrektur eines photometrischen Messwerts verwendet wird, wobei die Korrektur durch eine der folgenden Verfahren erfolgt:

a) Bereitstellen einer allgemeinen Formel zur Korrektur der Messwerte;
b) empirische Ermittlung einer Funktion zu Berechnung eines Korrekturfaktors, wobei in die Berechnung des Korrekturfaktors zumindest das tatsächlich pipettierte Volumen oder die Abweichung zum korrekten Wert eingehen;
c) empirische Ermittlung einer Schar von Funktionen zur Berechnung eines Korrekturfaktors , wobei in die Berechnung des Korrekturfaktors neben dem tatsächlich pipettierten Volumen auch die tatsächlich gemessene Extinktion eingeht; oder
d) empirische Ermittlung einer Korrekturmatrix, wobei mit Hilfe der tatsächlich ermittelten Extinktion und dem tatsächlich pipettierten Volumen bzw. der Abweichung vom korrekten Volumen der Korrekturfaktor abgelesen wird.

Ausführungsform 15: Photometer gemäß einem der Ausführungsformen 1 bis 14, dadurch gekennzeichnet, dass die Messung der flüssigen Probe anhand einer vorgelegten Reaktionslösung und einer durch Volumenkorrektur erfassten separat hinzugegebenen Probenlösung erfolgt und die Auswertung der Messergebnisse in der Datenverarbeitungseinheit eine Berechnung gemäß der folgenden Formel oder einer darauf basierenden Wertetabelle umfasst:

$$\frac{(Vr + Vprobe + \Delta V) \times Vprobe}{(Vprobe + \Delta V) \times (Vr + Vprobe)} \times E_{gemessen} = E_{korrigiert}$$

wobei $Vr$ das Volumen der vorgelegten Reaktionslösung, $Vprobe$ das geforderte Volumen der Probenlösung, $\Delta V$ der gemäß quantitativer Volumenerfassung ermittelte Volumenfehler der Probe als Abweichung von dem geforderten Volumen $Vprobe$, $E_{gemessen}$ die photometrisch ermittelte Extinktion und $E_{korrigiert}$ die korrigierte Extinktion ist.

Ausführungsform 16: Photometer gemäß einem der Ausführungsformen 1 bis 15, dadurch gekennzeichnet, dass das Photometer zusätzlich eine Anzeigeeinrichtung zur Darstellung der Messdaten und berechneten Werte und bevorzugt der korrigierten Probenkonzentration aufweist.

Ausführungsform 17: Photometer gemäß einem der Ausführungsformen 1 bis 16, dadurch gekennzeichnet, dass das Photometer ausgewählt ist aus der Gruppe enthaltend Spektralphotometer, Filterphotometer, LED Photometer und bevorzugt hierbei ein portables Photometer.

Ausführungsform 18: Verfahren zur photometrischen Konzentrationsbestimmung von mindestens einem Probenbestandteil in einer flüssigen Probe, bevorzugt durchgeführt mit einem Photometer gemäß einem der Ausführungsformen 1 bis 17, umfassend die folgenden Schritte:

a) Vorlegen einer Reaktionslösung in der Messküvette mit dem Volumen Vr;

b) Optional die quantitative Erfassung des Gewichts oder des Volumens der Reaktionslösung als Blindwert, bevorzugt im Photometer durchgeführt;

c) Zugabe der Probenlösung und Vermischung mit der Reaktionslösung;

d) Optional eine Dichtekorrektur, optional als Funktion der Volumendifferenz;

e) Quantitative Erfassung des Flüssigkeitsvolumens der Lösung aus Schritt c) mit Hilfe einer Wägevorrichtung, sonographischen oder optischen Messvorrichtung oder Bildaufzeichnungsvorrichtung, bevorzugt innerhalb eines Photometers;

f) Photometrische Bestimmung der Konzentration der Probe in der Lösung aus Schritt c).

g) Berechnung einer korrigierten Konzentration anhand des in Schritt e) quantitativ erfassten Volumens.

Ausführungsform 19: Verfahren zur photometrischen Konzentrationsbestimmung in einer flüssigen Probe, gemäß Ausführungsform 18, wobei die Berechnung der korrigierten Konzentration anhand der folgenden Formel durchge-führt wird:

$$\frac{(Vr + Vprobe + \Delta V) \times Vprobe}{(Vprobe + \Delta V) \times (Vr + Vprobe)} \times E_{gemessen} = E_{korrigiert}$$

wobei $Vr$ das Volumen der vorgelegten Reaktionslösung, $Vprobe$ das geforderte Volumen der Probenlösung, $\Delta V$ der Volumenfehler der Probe, $E_{gemessen}$ die photometrisch ermittelte Extinktion und $E_{korrigiert}$ die korrigierte Extinktion ist.

**Definitionen**

Gemäß der Erfindung ist unter einem Photometer ein Instrument zur Messung photometrischer Größen zu verstehen. Dies kann neben Messung im sichtbaren Bereich des Lichts (400 nm bis 800 nm) auch Messungen im UV-Bereich und/oder Infrarotbereich umfassen. Als photometrische Größe kann hier die Extinktion, Fluoreszenz, Polarisation oder Reflexion einer Probe vermessen werden.

Unter einer Messküvette ist erfindungsgemäß ein Behälter zu verstehen, der die zu vermessende Flüssigkeit enthält und für die Messung der photometrischen Größe im Photometer geeignet ist. Diese Messküvette ist aus einem Material und einer Oberflächenbeschaffenheit, die im Wellenlängenbereich, der bei der Messung zur Anwendung kommt, zweckmäßigerweise stark lichtdurchlässig bzw. transparent ist. In bevorzugter Weise handelt es sich bei der Messküvette um ein Gefäß mit planparallelen Seitenflächen. Es können aber auch Rundgefäße wie Reagenzgläser als Messküvette verwendet werden.

Unter einer Küvettenaufnahme ist im Rahmen der Erfindung eine Halterung zu verstehen, die die Messküvette in kontrollierter Weise aufnimmt, so dass sie im Strahlengang des Photometers positioniert ist.

Unter einer Messflüssigkeit ist die im Photometer zur Messung anstehende Flüssigkeit zu verstehen. In einer bevorzugten Weise handelt es sich hierbei um ein Gemisch aus einer Probenlösung und einer Reaktionslösung. Die Probenlösung ist diejenige Lösung, für die eine Konzentration mindestens eines Probenbestandteils photometrisch quantitativ bestimmt werden soll. Durch Umsetzung der Probenlösung mit der Reaktionslösung entsteht mindestens ein photometrisch detektierbares Reaktionsprodukt, das in seiner Konzentration Rückschlüsse auf die Konzentration des jeweiligen Probenbestandteils erlaubt und im Idealfall der Konzentration des Probenbestandteils entspricht. Die Umsetzung jegliche chemische Reaktionen umfassen, wie beispielsweise Säure-Basen-Reaktion, Komplexierung, Chelatisierung, Oxidation, Reduktion, Derivatisierung, Oligomerisierung, Polymerisierung oder Spaltung.

**Ausführungsbeispiele**

**1. Volumenkorrektur bei der CSB Bestimmung**

In diesem Ausführungsbeispiel wurde ein kommerzieller CSB 160 Test (MACHEREY-NAGEL, REF 985026) und ein kommerzielles Photometer (MACHEREY-NAGEL, NANOCOLOR Vis, REF 919150) verwendet. Das Standardprobenvolumen ist zu 2 mL spezifiziert. Dieses wurde im Beispiel um +/- 20% und +/- 10% variiert. Eingesetzte Volumina (als Abweichung vom Standardvolumen): -0,4 mL, -0,2 mL, 0 mL, 0,2 mL und 0,4 mL. Als Probe wurde ein CSB Standard mit Nennwert 114 mg/L $O_2$ eingesetzt.

Die Ergebnisse sind in der folgenden Tabelle dargestellt:

| 1 | 2 | 3 | 4 | 5 | 6 | 7 |
|---|---|---|---|---|---|---|
| Abweichung Probevol. vom Nennwert | Abweichung Probevol. in % | Meßwert CSB mg/L 02 | empirischer Faktor | Faktor aus der Funktion | Korrigierter Messwert | Abweichung in % |
| -0,4 | -20 | 76,1 | 0,6734513 | 1,4828 | 112,8 | -0,14 |
| -0,2 | -10 | 96,6 | 0,8548673 | 1,1751 | 113,5 | 0,46 |
| 0 | 0 | 113 | 1 | 0,9945 | 112,4 | -0,55 |
| 0,2 | 10 | 129 | 1,1415929 | 0,8787 | 113,4 | 0,31 |
| 0,4 | 20 | 141 | 1,2477876 | 0,8009 | 112,9 | -0,06 |

Die Spalte 1 bezeichnet hierbei die Abweichung des Probenvolumens vom Nennvolumen (2 mL). Die Spalte 2 bezeichnet die Abweichung des Probenvolumens vom Nennvolumen in %. In der Spalte 3 sind die mit dem Photometer nicht korrigierten Messwerte dargestellt. Da bei diesem Test ein umgekehrt proportionaler Zusammenhang zwischen dem photometrischen Messwert und der CSB Konzentration herrscht, steigt mit zunehmender Verdünnung der Probe auch der Meßwert CSB. Man sieht, dass der Volumenfehler zu einer deutlichen Abweichung der Messwerte vom Sollwert (hier 113 mg/L $O_2$ CSB) führt.

In diesem Beispiel wurde dann der Messwert (Spalte 3) durch den richtigen Messwert (Spalte 3, 113 mg/L $O_2$) geteilt und ein empirischer Faktor ermittelt. Anhand der Messwerte wurde eine Funktion ermittelt (polynomische Regression, siehe Fig. 7). Anschließend wurde mit der polynomischen Funktion und den Abweichungen des Probenvolumens (Spalte 1) ein Faktor aus der Funktion berechnet (Spalte 5). Dieser Faktor spiegelt die Volumenabhängigkeit der Messwerte wieder. In Spalte 6 sind die um diesen Faktor korrigierten Messwerte aus Spalte 3 dargestellt. Die prozentuale Abweichung vom Sollwert (Spalte 3, 113 mg/L 02 bei Nennvolumen) ist in Spalte 7 gezeigt. Nach Volumenkorrektur schwanken die einzelnen Messwerte nur um weniger als 1 %, obwohl die Volumenabweichung bis zu +/- 20 % betrug und die nicht korrigierten Messwerte um bis zu 30 % vom Sollwert abwichen.

Die Erfindung ist zudem in den folgenden Zeichnungen dargestellt und nachfolgend beschrieben.

Es zeigen:

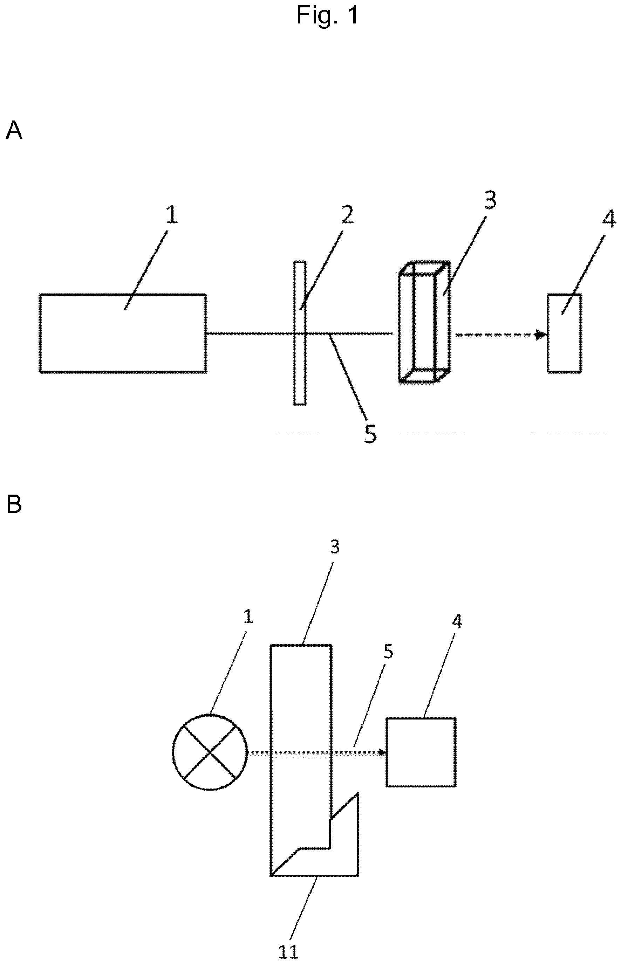

Fig. 1     eine übliche Anordnung bei einer photometrischen Messung mit einer Lichtquelle 1, einem Filter 2, der den Lichtstrahl 5 filtert, der im Strahlengang gelegenen Messküvette 3 und dem Detektor 4, in Fig. 1B die Grundbestandteile des Photometers mit Lichtquelle 1, einer im Strahlengang 5 gelegenen durch die Küvettenaufnahme 11 positionierten Messküvette 3 und dem Detektor 4.

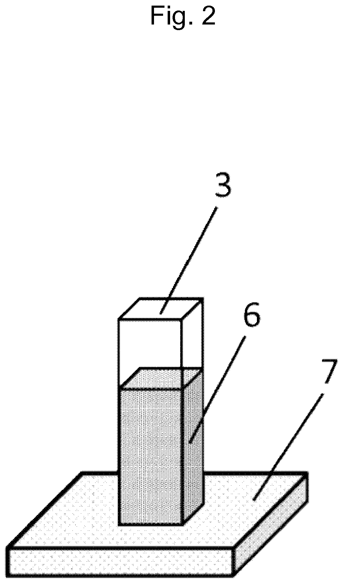

Fig. 2:     die Erfassung des Füllvolumens der Messküvette durch Wiegen der mit der Messflüssigkeit 6 gefüllten Messküvette 3, wobei die Messküvette 3 innerhalb des Photometers auf einer Wägeplattform 7 steht.

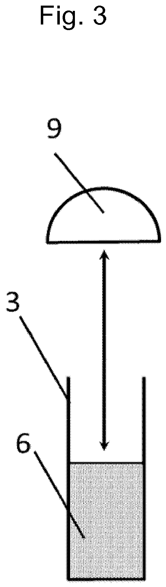

Fig. 3:     die Erfassung des Füllvolumens der Messküvette durch sonographische Messung der Füllhöhe der Messflüssigkeit 6 über eine oberhalb der Messküvette 3 angebrachte Ultraschallsonde 9.

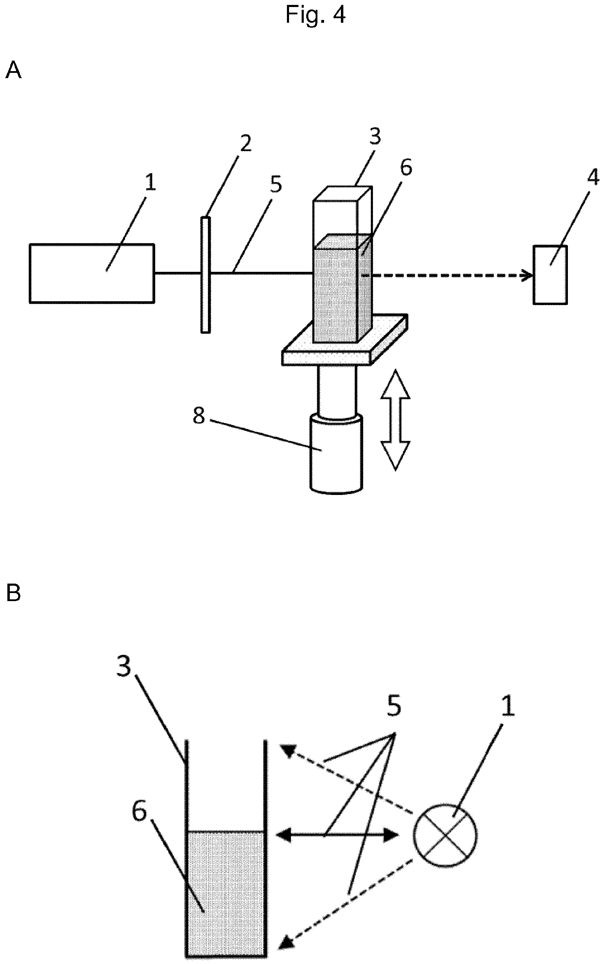

Fig. 4:     die Erfassung des Füllvolumens der Messküvette durch optische Messung der Füllhöhe der Messflüssigkeit 6 in (**A**) durch Vertikalbewegung der Messküvette 3 im Strahlengang 5 mittels eines Aktuators 8 und in (**B**) durch seitliches Bestrahlen der Küvette mit Licht und anschließender Detektion der reflektierten und/oder transmittierten Lichtstrahlen.

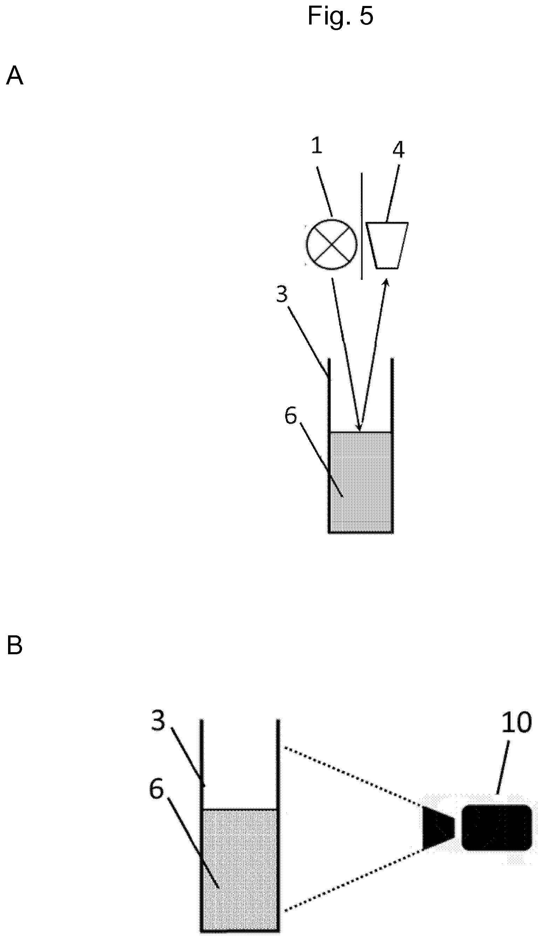

Fig. 5:     die Erfassung des Füllvolumens der Messküvette durch optische Messung der Füllhöhe der Messflüssigkeit 6 in (**A**) durch eine oberhalb der Messküvette 3 angeordneten Lichtquelle mit einem Detektor 4 zur Erfassung des reflektierten Lichts und in (**B**) durch Aufnahme der Küvette mit einer Fotokamera.

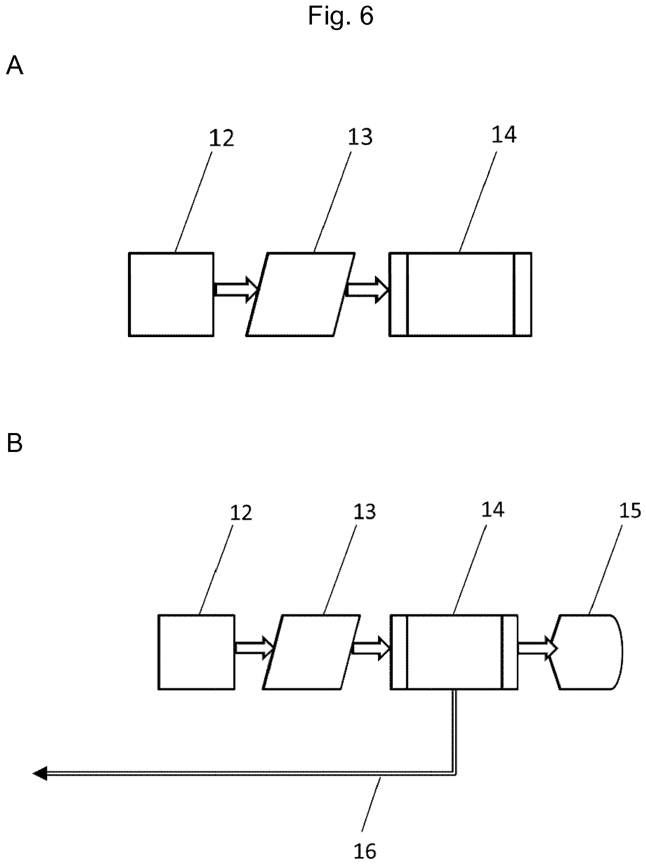

Fig. 6:     Schaltbilder für das erfindungsgemäße Photometer, wobei in (**A**) die Messeinheit 12 über einen Messdatenübertragung 13 (die beispielsweise einen A/D-Wandler beinhalten kann) an die Datenverarbeitungseinheit 14 sendet. In **(B)** ist an die Datenverarbeitungseinheit 14 noch eine Anzeigeeinheit 15 gekoppelt und die DVE 14 kann über eine Stellgröße 16 relevante Komponenten des Photometers, wie Waage, Lichtquelle, Filtereinheit oder Aktuator ansteuern.

Fig. 7: Volumenkorrektur bei der CSB-Bestimmung. Abbildung der Abhängigkeit des Volumenfehlers von der Volumendifferenz. Darstellung der Ergebnisse aus dem Ausführungsbeispiel 1.

**Bezugszeichenliste**

| | |
|---|---|
| 1 | Lichtquelle |
| 2 | Filter |
| 3 | Messküvette |
| 4 | Detektor |
| 5 | Lichtstrahl |
| 6 | zu vermessende Lösung (Messflüssigkeit) |
| 7 | Wägeplattform |
| 8 | Aktuator zur Vertikalbewegung der Messküvette |
| 9 | Ultraschallsonde |
| 10 | Fotokamera als Bildaufzeichnungseinrichtung |
| 11 | Küvettenaufnahme |
| 12 | Messeinheit |
| 13 | Messdatenübertragung |
| 14 | Datenverarbeitungseinheit |
| 15 | Anzeigeeinheit |
| 16 | Stellgröße für Waage, Lichtquelle, Filtereinheit oder Aktuator |

Weitere Varianten der Erfindung und ihre Ausführung ergeben sich für den Fachmann aus der vorangegangenen Offenbarung, den Figuren und den Patentansprüchen.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

**Patentansprüche**

**1.** Photometer zur photometrischen Konzentrationsmessung einer Messflüssigkeit in einer Messküvette umfassend eine Küvettenaufnahme und eine Wägevorrichtung zum Wiegen der Messküvette zur quantitativen Erfassung des Flüssigkeitsvolumens innerhalb der Messküvette.

**2.** Photometer gemäß Anspruch 1, **dadurch gekennzeichnet, dass** die quantitative Erfassung des Flüssigkeitsvolumens durch die Wägevorrichtung anhand der folgenden Formel erfolgt:

$$V_F = \frac{M_F}{\rho}$$

Wobei **$V_F$** das Flüssigkeitsvolumen, **$M_F$** die durch die Wägevorrichtung gemessene Masse und $\rho$ die Dichte der vermessenen Flüssigkeit ist, die bevorzugt gleich 1,0 g cm$^{-3}$ gesetzt wird, und wobei bei Zugabe der zu vermessenen Flüssigkeit zu einer vorgelegten Flüssigkeit mit stark abweichender Dichte im Photometer bevorzugt die Dichte korrigiert wird oder ein Dichteoffset hinterlegt ist, um zusätzlich eine Dichteoffsetkorrektur als Funktion der Volumendifferenz durchzuführen.

**3.** Photometer gemäß Anspruch 2, **dadurch gekennzeichnet, dass** das Gewicht oder das Volumen der vorgelegten Flüssigkeit als Blindwert vor Zugabe der zu vermessenen Flüssigkeit entweder gemessen wird und zur Korrektur des Blindwertes dient oder als Blindwert im Photometer hinterlegt ist.

4. Photometer gemäß einem der vorangehenden Ansprüche, **dadurch gekennzeichnet, dass** die Wägevorrichtung zum Wiegen der Messküvette ausgewählt ist aus der Gruppe enthaltend elektronische Waage, elektromechanische Waage, elektrische Waage, magnetische Waage und Präzisionswaage.

5. Photometer gemäß einem der vorangehenden Ansprüche, **dadurch gekennzeichnet, dass** bei dem Photometer die quantitative Erfassung des Flüssigkeitsvolumens an der in der Küvettenaufnahme befindlichen Messküvette vorgenommen wird.

6. Photometer gemäß einem der vorangehenden Ansprüche, **dadurch gekennzeichnet, dass** die Wägevorrichtung ein Bestandteil der Küvettenaufnahme ist.

7. Photometer gemäß einem der vorangehenden Ansprüche, **dadurch gekennzeichnet, dass** das Photometer zusätzlich eine Datenverarbeitungseinheit zur Auswertung der Messergebnisse der Messvorrichtung aufweist.

8. Photometer gemäß einem der vorangehenden Ansprüche **dadurch gekennzeichnet dass** die Bestimmung des Flüssigkeitsvolumen zur Korrektur eines photometrischen Messwerts verwendet wird, wobei die Korrektur durch eine der folgenden Verfahren erfolgt:

   a) Bereitstellen einer allgemeinen Formel zur Korrektur der Messwerte;
   b) empirische Ermittlung einer Funktion zu Berechnung eines Korrekturfaktors, wobei in die Berechnung des Korrekturfaktors zumindest das tatsächlich pipettierte Volumen oder die Abweichung zum korrekten Wert eingehen;
   c) empirische Ermittlung einer Schar von Funktionen zur Berechnung eines Korrekturfaktors , wobei in die Berechnung des Korrekturfaktors neben dem tatsächlich pipettierten Volumen auch die tatsächlich gemessene Extinktion eingeht; oder
   d) empirische Ermittlung einer Korrekturmatrix, wobei mit Hilfe der tatsächlich ermittelten Extinktion und dem tatsächlich pipettierten Volumen bzw. der Abweichung vom korrekten Volumen der Korrekturfaktor abgelesen wird.

9. Photometer gemäß einem der vorangehenden Ansprüche, **dadurch gekennzeichnet, dass** die Messung der flüssigen Probe anhand einer vorgelegten Reaktionslösung und einer durch Volumenkorrektur erfassten separat hinzugegebenen Probenlösung erfolgt und die Auswertung der Messergebnisse in der Datenverarbeitungseinheit eine Berechnung gemäß der folgenden Formel oder einer darauf basierenden Wertetabelle umfasst:

$$\frac{(Vr + Vprobe + \Delta V) \times Vprobe}{(Vprobe + \Delta V) \times (Vr + Vprobe)} \times E_{gemessen} = E_{korrigiert}$$

wobei *Vr* das Volumen der vorgelegten Reaktionslösung, *Vprobe* das geforderte Volumen der Probenlösung, $\Delta V$ der gemäß quantitativer Volumenerfassung ermittelte Volumenfehler der Probe als Abweichung von dem geforderten Volumen *Vprobe, $E_{gemessen}$* die photometrisch ermittelte Extinktion und *$E_{korrigiert}$* die korrigierte Extinktion ist.

10. Photometer gemäß einem der vorangehenden Ansprüche, **dadurch gekennzeichnet, dass** das Photometer zusätzlich eine Anzeigeeinrichtung zur Darstellung der Messdaten und berechneten Werte und bevorzugt der korrigierten Probenkonzentration aufweist.

11. Photometer gemäß einem der vorangehenden Ansprüche, **dadurch gekennzeichnet, dass** das Photometer ausgewählt ist aus der Gruppe enthaltend Spektralphotometer, Filterphotometer, LED Photometer und bevorzugt hierbei ein portables Photometer.

12. Verfahren zur photometrischen Konzentrationsbestimmung von mindestens einem Probenbestandteil in einer flüssigen Probe, bevorzugt durchgeführt mit einem Photometer gemäß einem der vorangehenden Ansprüche durchgeführt, umfassend die folgenden Schritte:

a) Vorlegen einer Reaktionslösung in der Messküvette mit dem Volumen Vr;

b) Optional die quantitative Erfassung des Gewichts oder des Volumens der Reaktionslösung als Blindwert, bevorzugt im Photometer durchgeführt;

c) Zugabe der Probenlösung und Vermischung mit der Reaktionslösung;

d) Optional eine Dichtekorrektur, optional als Funktion der Volumendifferenz;

e) Quantitative Erfassung des Flüssigkeitsvolumens der Lösung aus Schritt c) mit Hilfe einer Wägevorrichtung, bevorzugt innerhalb eines Photometers;

f) Photometrische Bestimmung der Konzentration der Probe in der Lösung aus Schritt c).

g) Berechnung einer korrigierten Konzentration anhand des in Schritt e) quantitativ erfassten Volumens.

13. Verfahren zur photometrischen Konzentrationsbestimmung in einer flüssigen Probe, gemäß Anspruch 12, wobei die Berechnung der korrigierten Konzentration anhand der folgenden Formel durchgeführt wird:

$$\frac{(Vr + Vprobe + \Delta V) \times Vprobe}{(Vprobe + \Delta V) \times (Vr + Vprobe)} \times E_{gemessen} = E_{korrigiert}$$

wobei $Vr$ das Volumen der vorgelegten Reaktionslösung, $Vprobe$ das geforderte Volumen der Probenlösung, $\Delta V$ der Volumenfehler der Probe, $E_{gemessen}$ die photometrisch ermittelte Extinktion und $E_{korrigiert}$ die korrigierte Extinktion ist.

# Fig. 1

A

B

Fig. 2

Fig. 3

# Fig. 4

A

B

# Fig. 5

A

B

# Fig. 6

A

B

**EUROPÄISCHER RECHERCHENBERICHT**

Europäisches Patentamt
European Patent Office
Office européen des brevets

Nummer der Anmeldung

EP 17 17 2345

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/107752 A1 (FERNANDO C J ANTHONY [US]) 6. Mai 2010 (2010-05-06) * Absätze [0022], [0023], [0026], [0027], [0034], [0039] * ----- | 1-7,10, 11 | INV. G01N21/59 G01N21/78 G01N33/18 G01F23/20 |
| X | WO 2005/050183 A2 (NTTF GMBH [DE]; BUSCH HEINZ WERNER [DE]; GRABOWY UDO HEINRICH [DE]; TH) 2. Juni 2005 (2005-06-02) * Seite 2, Zeile 18 - Zeile 27 * * Seite 5, Zeile 4 - Zeile 8 * * Seite 19, Zeile 30 - Seite 20, Zeile 27 * * Seite 14, Zeile 1 - Zeile 6 * * Abbildung 2 * ----- | 1,2,4-7, 10,11 | ADD. G01N21/11 |
| X | US 4 144 030 A (SUOVANIEMI OSMO) 13. März 1979 (1979-03-13) | 1-5,7-13 | |
| Y | * Spalte 2, Zeile 38 - Spalte 3, Zeile 22 * * Spalte 4, Zeile 48 - Zeile 58 * * Spalte 5, Zeile 20 - Spalte 6, Zeile 40 * * Abbildung 1 * ----- | 8,9,12, 13 | |
| X | JP H06 109642 A (NAKANO VINEGAR CO LTD) 22. April 1994 (1994-04-22) * Zusammenfassung * * Absätze [0014], [0016], [0020] * * Abbildung 1 * ----- | 1-7,10, 11 | RECHERCHIERTE SACHGEBIETE (IPC) G01N G01F |
| Y | EP 0 497 343 A2 (SANYO ELECTRIC CO [JP]) 5. August 1992 (1992-08-05) * Spalte 3, Zeile 3 - Zeile 27 * ----- -/-- | 8,9,12, 13 | |

Der vorliegende Recherchenbericht wurde für alle Patentansprüche erstellt

| Recherchenort | Abschlußdatum der Recherche | Prüfer |
|---|---|---|
| München | 22. September 2017 | D'Alessandro, Davide |

EPO FORM 1503 03.82 (P04C03)

**Europäisches Patentamt**
**European Patent Office**
**Office européen des brevets**

## EUROPÄISCHER RECHERCHENBERICHT

Nummer der Anmeldung

EP 17 17 2345

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | B. W. RENOE ET AL: "Automated computer-controlled solution handling system utilizing weights of solution", ANALYTICAL CHEMISTRY, Bd. 48, Nr. 4, 1. April 1976 (1976-04-01), Seiten 661-666, XP55408456, US ISSN: 0003-2700, DOI: 10.1021/ac60368a040 * Zusammenfassung * * Seite 661, linke Spalte, letzter Absatz * * Seite 664, rechte Spalte, Absatz 1 * * Seite 665, rechte Spalte, letzter Absatz - Seite 666, linke Spalte * ----- | 12 | |
| A | DE 30 30 103 A1 (GAT GROVE ANALYSENTECHNIK GMBH [DE]) 11. März 1982 (1982-03-11) * Absätze [0006], [0009] * ----- | 1-13 | |
| A | US 2010/118130 A1 (HARRIS J KEITH [US] ET AL) 13. Mai 2010 (2010-05-13) * Absätze [0028], [0038] * * Abbildungen 2,3 * ----- | 1-11 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 22. September 2017 | D'Alessandro, Davide |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 17 17 2345

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-09-2017

| Im Recherchenbericht angeführtes Patentdokument | Datum der Veröffentlichung | Mitglied(er) der Patentfamilie | Datum der Veröffentlichung |
|---|---|---|---|
| US 2010107752 A1 | 06-05-2010 | US 2010107752 A1<br>WO 2010056525 A2 | 06-05-2010<br>20-05-2010 |
| WO 2005050183 A2 | 02-06-2005 | AT 371859 T<br>CA 2545643 A1<br>EP 1685388 A2<br>ES 2293367 T3<br>MY 135482 A<br>WO 2005050183 A2 | 15-09-2007<br>02-06-2005<br>02-08-2006<br>16-03-2008<br>30-04-2008<br>02-06-2005 |
| US 4144030 A | 13-03-1979 | FI 762239 A<br>US 4144030 A | 05-02-1978<br>13-03-1979 |
| JP H06109642 A | 22-04-1994 | KEINE | |
| EP 0497343 A2 | 05-08-1992 | DE 69215394 D1<br>DE 69215394 T2<br>EP 0497343 A2<br>JP H04248447 A<br>US 5296194 A | 09-01-1997<br>26-06-1997<br>05-08-1992<br>03-09-1992<br>22-03-1994 |
| DE 3030103 A1 | 11-03-1982 | KEINE | |
| US 2010118130 A1 | 13-05-2010 | KEINE | |

EPO FORM P0461

Für nähere Einzelheiten zu diesem Anhang : siehe Amtsblatt des Europäischen Patentamts, Nr.12/82